# EUROPEAN PATENT SPECIFICATION

(11) **EP 0 973 780 B1**
(45) Date of publication and mention of the grant of the patent: **02.07.2003**
(21) Application number: 98922631.1
(22) Date of filing: 01.04.1998
(51) Int. Cl.: C07D 501/38, C07D 295/20, A61K 31/545

(54) **ANTIBACTERIAL SUBSTITUTED 7-ACYLAMINO -3-(METHYLHYDRAZONO) METHYL-CEPHALOSPORINS AND INTERMEDIATES**
ANTIBAKTERIELLE SUBSTITUIERTE 7-ACYLAMINO-3-(METHYLHYDRAZONO)METHYL-CEPHALOSPORINE UND ZWISCHENPRODUKTE
CEPHALOSPORINES ANTIBACTERIENNES DE 7-ACYLAMINO -3-METHYLHYDRAZONOMETHYLE SUBSTITUE ET INTERMEDIAIRES

(30) Priority: 01.04.1997 AT 54697; 01.04.1997 AT 54797; 01.04.1997 AT 54897
(43) Date of publication of application: 26.01.2000
(60) Divisional application: 03000220.8
(73) Proprietor: BIOCHEMIE GESELLSCHAFT M.B.H., 6250 Kundl (AT)
(72) Inventor: ASCHER, Gerd, A-6250 Kundl (AT); WIESER, Josef, A-6403 Polling (AT); SCHRANZ, Michael, A-1200 Wien (AT); LUDESCHER, Johannes, A-6252 Breitenbach (AT); HILDEBRANDT, Johannes, A-2512 Oeynhausen (AT)
(74) Representative: Grubb, Philip William
(86) International application number: EP9801890
(87) International publication number: WO98043981

(56) References cited:
- WO-A-96/35692
- GB-A- 2 151 617

## Description

The present invention relates to antimicrobial cephalosporins. WO96/35692 discloses antibacterial effective cephalosporin compounds wherein the 7-position is substituted by a 2-(2-aminothiazol-4-yl)-alkoxyiminoacetamido- or a 2-(2-aminothiadiazol-4-yl)alkoxyiminoacetamido group and the 3-position is substituted by e.g. a [((heterocycle)iminomethyl)hydrazono]methyl group. Inter alia a compound of formula 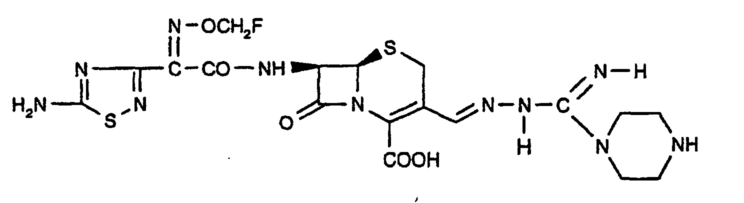 is disclosed.

The present invention provides in one aspect a compound of formula 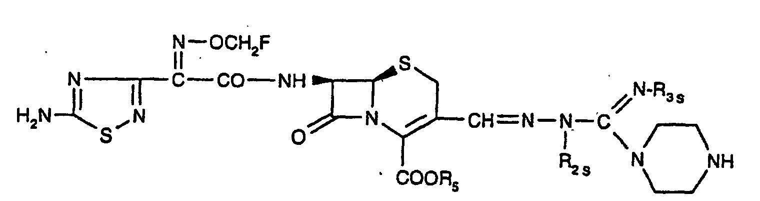 wherein
R₅ denotes hydrogen, (C₁₋₆)alkyl, benzyl, (C₁₋₈)alkoxybenzyl, indanyl, phtalidyl, (C₁₋₈)alkoxymethyl, (C₁₋₆)alkanoyloxy(C₁₋₆)alkyl, (C₁₋₆)alkoxycarbonyloxy(C₁₋₆)alkyl, glycyloxymethyl, phenylglycyloxymethyl or (5-methyl-2-oxo-1,3-dioxolen-4-yl)methyl ;
R₂ₛ denotes (C₁₋₆)alkyl, ar(C₁₋₆)alkyl, (C₂₋₆)alkenyl or (C₂₋₈)alkinyl, and
R₃ₛ denotes hydrogen, (C₁₋₆)alkyl, ar(C₁₋₆)alkyl, (C₂₋₆)alkenyl, (C₂₋₈)alkinyl or (C₃₋₈)cycloalkyl, in free form or in form of a salt or in form of a solvate.

Aryl includes aryl containing up to 18, e.g. 12 C atoms, including e.g. phenyl, napthyl. (C₃₋₈)cycloalkyl includes (C₃₋₆)cycloalkyl.

The ester-moiety R₅ is selected from C₁₋₆alkyl, e.g. C₁₋₄alkyl; benzyl; (C₁₋₈)alkoxybenzyl, such as 4-methoxybenzyl; indanyl; phthalidyl; (C₁₋₈)alkoxymethyl, e.g. methoxymethyl; (C₁₋₆)alkanoyloxy(C₁₋₆)alkyl; (C₁₋₆)alkoxy-carbonyl-oxy(C₁₋₆)alkyl; glycyloxymethyl; phenylglycyloxymethyl; or (5-methyl-2-oxo-1,3-dioxolen-4-yl)methyl. The ester moiety also includes ester moieties which form with the COO- group a physiologically hydrolysable and acceptable ester, e.g. such known to be hydrolysable ester groups in the field of cephalosporins. A compound of formula I may thus be in the form of an physiologically-hydrolysable and -acceptable ester. By physiologically-hydrolysable and -acceptable esters as used herein is meant an ester in which the COO- group is esterified and which is hydrolysable under physiological conditions to yield an acid which is itself physiologically tolerable at dosages to be administered. The term is thus to be understood as defining regular pro-drug forms. An ester moiety may be preferably a group which is easily hydrolysable under physiological conditions. Such esters may be administered preferably orally. Parenteral administration may be indicated if the ester per se is an active compound or, if hydrolysis occurs in the blood. A silyl group is a silyl protecting group, *e.g*. a conventional silyl protecting group, such as a trialkylsilyl group, for example the trimethylsilyl group. A leaving group includes e.g. a leaving group which is conventional in a type of reaction described; in an acylation reaction of an amine group e.g. a carboxylic acid derivative, such as a carboxylic acid halogenide, (active) ester, (mixed) anhydride) may be an appropriate acylation agent. A cation includes a cation which may form a pharmaceutically acceptable salt with a compound of formula I; e.g. a metal salt such as sodium, potassium; or an amine (ammonium) salt, such as trialkylamine, procain, dibenzylamine, benzylamine, ammonium salt.

A compound of formula Is includes a compound
wherein
R₅ is as defined above;
R₂ₛ and R₃ₛ independently of each other denote C₁₋₆alkyl, such as C₁₋₄ alkyl; ar (C₁₋₆)alkyl, such as ar(C₁₋₄)alkyl; (C₂₋₆)alkenyl, such as (C₂₋₄) alkenyl; or (C₂₋₈) alkinyl; and R₃ₛ additionally denotes hydrogen or (C₃₋₈) cycloalkyl; e.g. R₂ₛ denotes alkyl, alkenyl or aralkyl; e.g. R₃ₛ denotes hydrogen or alkyl; e.g. a compound of formula 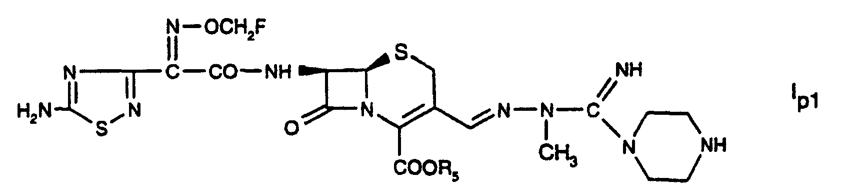 wherein R₅ is as defined above.

A compound of formula Is may be e.g.in free form and in the form of a salt and/or in the form of a solvate.
A salt includes any possible salt, e.g. an acid addition salt; such as a hydrochloride, internal salt, metal salt, quaternary salt and an amine salt of a compound of formula Is. Metal salts include for example sodium, potassium, calcium, barium, zinc, aluminum salts, preferably sodium or potassium salts. Amine salts include for example trialkylamine, procaine, dibenzylamine and benzylamine salts. A salt may preferably be a pharmaceutically acceptable salt of a compound of formula Is.

A solvate includes a solvate with an organic solvent and a solvate with water, such as a hydrate.
A compound of formula Is may be e.g. in the form of a hydrochloride, such as a monohydrochloride, dihydrochloride, trihydrochloride, e.g. in crystalline form and /or in the form of a solvate, e.g. a hydrate.
A free form of a compound of formula Is may be converted into a salt form and vice versa. A solvate form of a compound of formula Is, e.g. in free form or in the form of a salt, may be converted in a non-solvate form and vice versa.
A compound of formula Is includes a compound of formula Is in any configuration, e.g. in any possible steroisomeric form. Mixtures of stereoisomeric forms may be separated, e.g. as conventional, e.g. by chromatography, fractioned crystallisation. E.g. the configuration of R₁ in group -C=NOCH₂F may be syn [(Z)] and anti [(E)] and is preferably, e.g. predominantly, syn [(Z)]; e.g. containing the [(E)] form in an amount of 0 to 5%, e.g. 0 to 2%.
A compound of formula Is may be in the form of a mixture of the 3(E)-form and 3-(Z)-form, or may be, e.g. predominantly, in the 3(Z)-form, e.g. according to formula 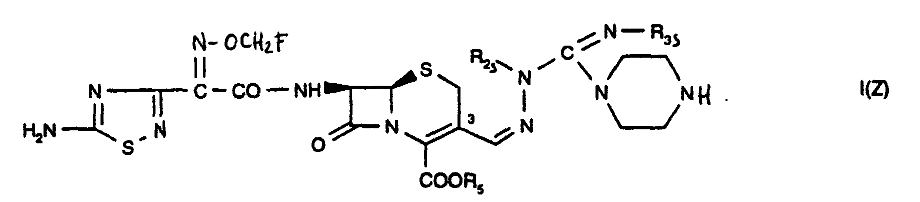 or may be, e.g. predominantly, in the 3(E)-form, e.g. according to formula 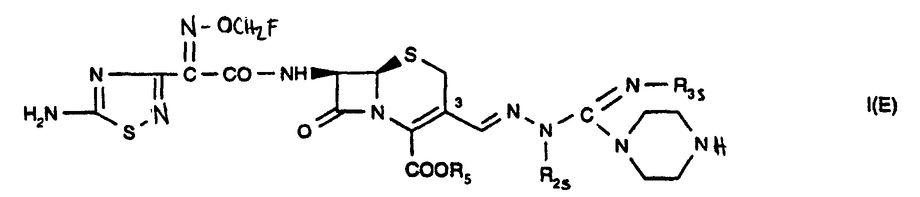 and wherein the configuration of the group 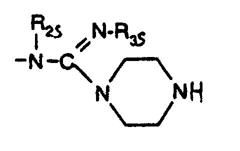 attached to the nitrogen of the -C=N group in position 3 of the ring system is, e.g. 3(E) and/or 3(Z). A compound of formula I may be, e.g. predominantly, in the 3(E)-form, e.g. containing the 3(Z)-form in an amount of 0 to 5%, e.g. 0 to 2% or predominantly in the 3(Z)-form , e.g. containing the 3(Z)-form in an amount of 0 to 5%, e.g. 0 to 2%. A compound of formulae Is and Iₚ₁ may be, e.g. predominantly, in the 3(E)-form, e.g. containing the 3(Z)-form in an amount of 0 to 5%, e.g. 0 to 2%.

A compound of formula Is may be obtained as follows:
Reacting a compound of formula 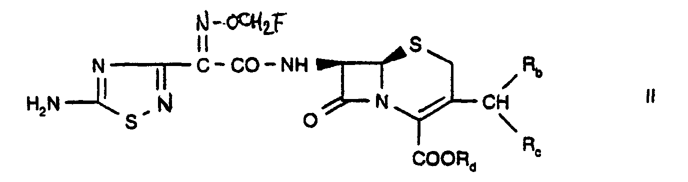 wherein W, V and R₁ are as defined above and wherein
   α) R_{b} denotes hydroxy and R_{c} and R_{d} together denote a bond, or
   β) R_{d} denotes hydrogen, a cation, an ester moiety R₅ or a silyl protecting group and R_{b} and R_{c} denote the oxo group
   e.g. in free form or in the form of an acid addition salt, with a compound of formula 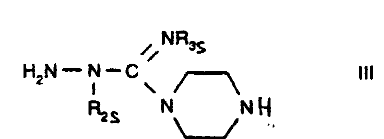
wherein R₂ₛ and R₃ₛ are as defined above, e.g. in free form or in the form of an acid addition salt, e.g. as appropriate, e.g. as conventional.

Reactive groups in a compound of formulae Is, II, III may be protected by protecting groups, e.g. protecting groups which are conventional, e.g. in cephalosporin chemistry. Silyl protecting group technology in the presence of a solvent which may be inert towards silylation agents, e.g. a chlorinated hydrocarbon, such as dichloromethane; a nitrile such as acetonitrile, an ether such as tetrahydrofuran, a dipolar aprotic solvent, e.g. N,N-dimethylformamide; or a solvent system, *e.g.* mixtures of individual solvents, *e.g*. as described above; may be appropriate for the protection of reactive groups. Protecting groups may be split off, e.g. as conventional during a corresponding reaction or after termination of a corresponding reaction. A compound of formula Is wherein R₅ denotes hydrogen may be converted into a compound of formula Is wherein R₅ denotes an ester moiety or vice versa. A compound of formula Is may be isolated from the reaction mixture, e.g. as conventional. A compound of formula Is may be obtained in free form or in the form of a salt and/or a hydrate. A compound of formula Is in free form may be converted into a compound of formula in the form of a salt and/or a hydrate and vice versa.

The process may be carried out as follows:
A compound of formula II may be reacted with a compound of formula III, e.g. in a solvent, e.g. in a solvent which is inert under the reaction conditions, such as water; a mixture of water with an e.g. lower, e.g. (C₁₋₄)alcohol or dioxane; or in a dipolar aprotic solvent, e.g. dimethylformamide, dimethylsulfoxide, dimethylacetamide, if desired in mixture with an alcohol and/or water; at temperatures from -20 to 50° C. The pH may be at an optimum, e.g. by addition of an organic or inorganic acid or base. A compound of formula Is obtained may be isolated and/or purified, e.g. as conventional, e.g. by addition of an anti-solvent or by chromatography.

Starting compounds are known or may be produced according to known, e.g. analogous methods, or e.g. according to the present examples. A part of the starting compounds according to the present invention is novel.

In another aspect the present invention provides a compound selected from
1-[(1-Ethylhydrazino)iminomethyl]piperazine
1-[(1-Allylhydrazino)iminomethyl]piperazine
1-[(1-(4-Methoxybenzyl)hydrazino]iminomethyl]piperazine
1-[(1-(3,4,5-Trimethoxybenzyl)hydrazino]iminomethyl]piperazine
1-[(1-Methylhydrazino)(methylimino)methyl]piperazine
e.g. in the form of a salt, such as a hydrochloride, and/or in the form of a solvate; and, in another aspect, a compound of formula 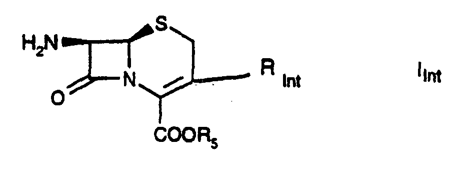 wherein R₅ is as defined in claim 1, and Rᵢₙₜ denotes a group 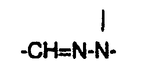 wherein the =N- group corresponds to the terminal amine group of the hydrazino group of a compound selected from the list above and wherein the -N- group is substituted according to a compound selected from the list above, i.e. a hydrazino-compound listed above is bond to the ring system via the terminal amine group of the hydrazino group to the methyl group in position 3 of the ring system to form a group 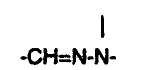 wherein the -N- group is substituted according to a hydrazino compound listed above.

The compounds of formulae Is, hereinafter designated as "active compound(s) of the invention" exhibits pharmacological activity and surprising low toxicity and are therefore useful as pharmaceuticals. In particular, the active compounds of the invention show antimicrobial, *e.g*. antibacterial, activity against aerobic and anaerobic growing bacteria, e.g. gram negative and gram positive bacteria such as *Enterobacter, e.g. Enterobacter cloacae; Enterococcus, e.g. Enterococcus faecalis, Enterococcus faecium; Moraxella, e.g. Moraxella catarrhalis; Haemophilus, e.g. Haemophilus influenza; Klebsiella, e.g. Klebsiella edwardsii, Klebsiella pneumoniae; Streptococcus, e.g. Streptococcus pyogenes; Staphylococcus, e.g.* *Staphylococcus aureus* MSSA (methicillin sensitive strains); *Staphylococcus aureus* MRSA (methicillin resistant strains); *Escherichia, e.g. Escherichia coli; Proteus, e.g. Proteus mirabilis, Salmonella, e.g. Salmonella typhimurium, Serratia, e.g. Serratia marcescens, Pseudomonas, e.g. Pseudomonas aeruginosa; Pneumococci, e.g. Pneumococcus pneumoniae* (penicillin sensitive and mult-drug resistant strains); in *vitro* in the Agar DilutionTest for bacteria according to National Commitee for Clinical Laboratory Standards (NCCLS) 1993,
- Document-M7-A3Vol.13, No. 25: "Methods for dilution Antimicrobial Susceptibility Tests for Bacteria that Grow Aerobically - Third Edition, Approved Standard"; and
- Document M11-A3 for anaerobic bacteria
in a concentration from about 0.001 to ca. 50 µg/ml (MIC), *e.g.* using strains including *Staphylococcus aureus* (ATCC 29213 and ATCC 9144); *Enterococcus faecalis* (ATCC 29212); *Haemophilus influenza* (NTCC 49247 and NCTC 11931); *Escherichia coli* (ATCC 25922 and ATCC 35218); *Klebsiella pneumoniae* (NCTC 11228); *Klebsiella edwardsii* (NCTC 10896); *Pseudomonas aeruginosa* (ATCC 27853 and ATCC 25668);
and *in vivo* in the septicaemia mouse model, in accordance to the method description Nr. 159 A-5, approved by Austrian Health Authorities (MA 58, no. 2968/95 of 12-Oct-1995), e.g. when administerd at dosages from about 0.05 to 50 mg/kg body weight, such as 0.1 to 50 mg/kg body weight (ED₅₀ values). E.g., mice are infected with an ED 95% of *Staphylococcus aureus* (ATCC 4995), *Streprococcus pyogenes* (ATCC 29218), *Escherichia coli* (Δ 12 NFI culture collection) and are treated 1, 5 and 24 hours after infection. The ED 50% values ranging from ca. 0.2 to 50 mg/kg body weight arc calculated by Probit analysis of the administered dosages of compounds. Activity is determined by numbers of surviving animals per group of 8 mice per dosage until day 5 after infection.
The active compounds of the invention show an surprising overall activity spectrum.
It has, for example, been determined that the MHK (µg/ml) of the compound of Example 1 against, for example *Enterococcus faecalis* is of ca. 0.1 to 0.4; against *Staphylococcus aureus* (MSSA) is of ca. < 0.125 to 0.8; against methicillin resistant *Staphyloccous aureus* is of 0.8 to 6.4; against multi-drug resistant *Pneumococcus* is of 0.4.
The active compounds of the invention are therefore useful for the treatment of microbial, e.g. bacterial diseases.

For this indication, the appropriate dosage will, of course, vary depending upon, for example, the compound of formula Is employed, the host, the mode of administration and the nature and severity of the conditions being treated. However, in general, for satisfactory results in larger mammals, for example humans, an indicated daily dosage is in the range from about 0.05 to 5 g, for example 0.1 to about 2.5 g, of an active compound of the invention conveniently administered, for example, in divided doses up to four times a day.

An active compound of the invention may be administered by any conventional route, for example orally, e.g. in the form of tablets or capsules, or parenterally in the form of injectable solutions or suspensions, e.g. in analogous manner to cefotaxime.

The compound 7-(((5-amino-1,2,4-thiadiazol-3-yl)-(Z)-(fluormethoxyimino)acetyl)amino)-3-((imino-1-piperazinylmethyl)methylhydrazono)methyl-3-cephem-4-carboxylic acid (compound of Example 1) is the preferred compound of the invention for use as an antimicrobial agent. It has, for example been determined that the MHK (µg/ml) of the compound of Example 1 (tested in the form of the hydrochloride) against, for example *Haemophilus influenza* is ca. < 0.125 to 0.4 and, for example cefotaxime shows an MHK (µg/ml) of ca. < 0.125 to 0.4. It is therefore, indicated that for the treatment of microbial diseases, e.g. bacterial diseases the preferred compounds of the invention may be administered to larger mammals, for example humans, by similar modes of administration at similar dosages than conventionally employed with cefotaxime.

A compound of formula Is may be administered in the form of a pharmaceutically acceptable salt, e.g. an acid addition salt or a base addition salt or in the corresponding free form, if desired in the form of a solvate. Such a salt/solvate may exhibit the same order of activity as the free form.

The present invention also provides a pharmaceutical composition comprising a compound of formula Is according to claim 1 in the form of a pharmaceutically acceptable salt or in free form in association with at least one pharmaceutical carrier or diluent.

Such compositions may be manufactured in conventional manner.
Unit dosage form may contain, for example 10 mg to about 1 g, for example 10 mg to about 700 mg, such as to about 500 mg.

As medicaments, the active ingredients according to the invention may be administered alone or in suitable medicinal forms together with inorganic or organic, pharmacologically inert excipients. For example, they are used as a constituent of capsules, or injection or instillation preparations, which contain a quantity of active compounds that is sufficient to attain an optimum blood level, that is, ca. 10 to 500 mg per capsule. For this application, the dosage to be administered depends on the compound used and the type of administration, as well as the type of treatment. With larger mammals, satisfactory results may be obtained when administering a daily dose of ca. 0.5 to 6 g. If required, this amount may be given in correspondingly smaller doses two to four times daily, or in sustained release form.

In another aspect the present invention provides a compound of formula Is or a composition comprising a compound of formula Is in the form of a pharmaceutically acceptable salt or in free form in association with at least one pharmaceutical carrier or diluent for use as a pharmaceutical, e.g. as an antibiotic; and
The use of a compound of formula Is, or use of a composition comprising a compound of formula Is in the form of a pharmaceutically acceptable salt or in free form in association with at least one pharmaceutical carrier or diluent as a pharmaceutical.

In a further aspect the present invention provides a method of treatment of microbial diseases, e.g. caused by bacterias selected from *Pseudomonas, Enterobacter, Enterococcus, Moraxella, Haemophilus, Klebsiella, Streptococcus, Staphylococcus, Escherichia, Proteus, Salmonella, Serratia* or *Pneumococci,* which comprises administering to a subject in need of such treatment, an effective amount of a compound of formula Is. e.g. in the form of a pharmaceutical composition according to the present invention; and
A compound of formula Is for use in the preparation of a medicament for the treatment of microbial diseases, for example of diseaeses caused by bacterias selected from *Pseudomonas, Enterobacter, Enterococcus, Moraxella, Haemophilus, Klebsiella, Streptococcus, Staphylococcus, Escherichia, Proteus, Salmonella, Serratia* or *Pneumococci*.

In the following examples, which illustrate the invention more fully but should in no way limit its scope, all temperatures are given in degrees Celsius. ¹H-NMR: 200MHz, DMSO-d₆.

### Example 1

### 7-(((5-amino-1,2,4-thiadiazol-3-yl)-(Z)-(fluoromethoxyimino)acetyl)amino)-3-(imino-1-piperazinylmethyl)methylhydrazono)methyl-3-cephem-4-carboxylic acid

### a) N-(1,4,5a,6-tetrahydro-3-hydroxy-1,7-dioxo-3H,7H-aceto(2,1-b)furo(3,4-d)(1,3)-thiazin-6-yl)-2-(5-amino-1,2,4-thiadiazol-3-yl)-(Z)-2-(fluoromethoxyimino)acetic acid amide (hydroxylactone of 7-(((5-amino-1,2,4-thiadiazol-3-yl)-(Z)-(fluoromethoxyimino)acetyl)amino)-3-formyl-3-cephem-4-carboxylic acid)

A suspension of 10 g of 7-amino-3-formyl-3-cephem-4-carboxylic acid in a mixture of 220 ml of methylene chloride and 80 ml of acetonitrile is stirred at 0° with 43 ml of N,O-bis(trimethylsilyl)-acetamide. 15.7 g of (5-amino-1,2,4-thiadiazol-3-yl)-(Z)-2-fluoromethoxyimino-acetic acid chloride are added to the clear solution obtained and the reaction mixture is stirred for ca. one hour at ca. 0°. The mixture is diluted with 1250 ml of acetonitrile which contains 70 ml of water. 12% aqueous ammonia is added to the mixture obtained to adjust a pH value of 3.5. The mixture is diluted with 2.5 litres of water and extracted with ethyl acetate. The ethyl acetate phase is dried and concentrated. The concentrate is stirred for one hour at 20° with 100 ml of acetonitrile. N-(1,4,5a,6-tetrahydro-3-hydroxy-1,7-dioxo-3H,7H-aceto(2,1-b)furo(3,4-d)(1,3)-thiazin-6-yl)-2-(5-amino-1,2,4-thiadiazol-3-yl)-(Z)-2-(fluoromethoxyimino)acetic acid amide precipitates in crytalline form, is filtrated off and dried.

### b) 7-(((5-amino-1,2,4-thiadiazol-3-yl)-(Z)-(fluoromethoxyimino)acetyl)amino)-3(E)-(imino-1-piperazinylmethyl)methylhydrazono)methyl-3-cephem-4-carboxylic acid

3.77 g of N-(1,4,5a,6-tetrahydro-3-hydroxy-1,7-dioxo-3H,7H-aceto(2,1-b)furo(3,4-d)(1,3)-thiazin-6-yl)-2-(5-amino-1,2,4-thiadiazol-3-yl)-(Z)-2-fluoromethoxyimino)acetic acid amide are suspended in a mixture of 75 ml of acetonitrile and 11 ml of water and treated with a solution of 2 g of 1-(1-methylhydrazino)iminomethyl)piperazine in the form of a dihydrochloride in 4.5 ml of 2N HCI. The reaction mixture is stirred for ca. one day at room temperature and poured into 600 ml of acetonitrile under stirring. 7-(((5-amino-1,2,4-thiadiazol-3-yl)-(Z)-(fluoromethoxy-imino)acetyl)amino)-3(E)-(imino-1-piperazinylmethyl)-methylhydrazono)methyl-3-cephem-4-carboxylic acid in the form of a trihydrochloride precipitates, is filtrated off, washed with acetonitrile and dried.

### c) 7-(((5-amino-1,2,4-thiadiazol-3-yl)-(Z)-(fluoromethoxyimmo)acetyl)amino)-3(E)-(imino-1-piperazinylmethyl)methylhydrazono)methyl-3-cephem-4-carboxylic acid

0.65 g of crude 7-(((5-amino-1,2,4-thiadiazol-3-yl)-(Z)-(fluoromethoxy-imino)acetyl)amino)-3(E)-(imino-1-piperazinylmethyl)methylhydrazono)methyl-3-cephem-4-carboxylic acid in the form of a trihydrochloride obtained in step b) are dissolved in 2 ml of water and filled into a column which is filled with 50 g of RP-18^{R} (LiChroprep RP-18^{R}, grain size 40-63 µm, Merck) and eluated with water (flow rate 20ml/min). Fractions are examined by means of analytical HPLC and the fractions which contain 7-(((5-amino-1,2,4-thiadiazol-3-yl)-(Z)-(fluoromethoxyimino)acetyl)-amino)-3(E)-(imino-1-piperazinylmethyl)methylhydrazono)methyl-3-cephem-4-carboxylic acid in the form of a monohydrochlorid are determined (HPLC), combined and lyophilised.

In the manner described in Example 1 but using corresponding compounds of formulae II and III wherein R₂ₛ, R₃ₛ and R₅ have the meaning given in TABLE 1 below, compounds of formula Is, wherein R₅ = H and R₂ₛ and R₃ₛ have the meaning listed in TABLE 1 below are obtained, e.g. in the salt form described:

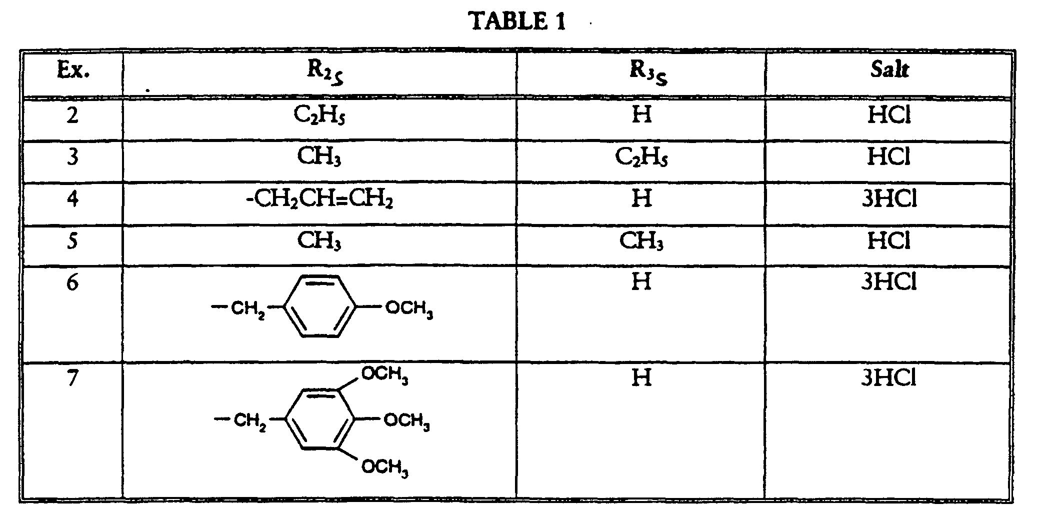

Compounds useful as starting material according to the present invention may e.g. be produced as follows:

### Example A

### 1-(1-Methylhydrazino)iminomethyl)piperazine

### a) S-Methvl-2-methyl-isothiosemicarbazide

A solution of 239.8 g of S-methyl-2-methylisothio-semicarbazide in the form of a hydriodide in 100 ml of water is placed on a column filled with 1500 ml of a strong basic ion exchanger in chloride form (Amberlite IRA 420^{R}), and eluted with water. The fractions containing S-methyl-2-methylisothio-semi-carbazide in the form of a hydrochloride (HPLC) are lyophilised. The lyophilisate is treated with ether, isolated by filtration and dried.
S-methyl-2-methyl-isothiosemicarbazide in the form of a hydrochloride is obtained in the form of a white solid.
M.p.: 116° (isopropanol).

### b) Benzylidene derivative of 4-formyl-1-((1-methylhydrazino)imino-methyl)piperazine

A solution of 40.9 g of S-methyl-2-methyl-isothiosemicarbazide in the form of a hydrochloride in 350 ml of ethanol is mixed with 30 g of freshly distilled formylpiperazine and heated under reflux for ca. 39 hours. The reaction mixture is cooled to room temperature, mixed with 26.4 ml of benzaldehyde and stirred for ca. 24 hours. The precipitate obtained is filtrated off, washed with ethanol and dried. The benzylidene derivative of 4-formyl-1-((1-methylhydrazino)imino-methyl)piperazine in the form of a hydrochloride is obtained.

### c) 1-((1-Methylhydrazino)iminomethyl)piperazine

From 10 g of the benzylidene derivative of 4-formyl-1-((1-methylhydrazino)iminomethyl)piperazine in the form of a hydrochloride the benaldehyde is split off by steam distillation under addition of 48 ml of 2N HCl. The aqueous slurry obtained is concentrated and an oily residue is obtained which is treated with boiling ethanol. The ethanolic phase is concentrated in vacuum. 1-((1-Methylhydrazino)iminomethyl)piperazine in the form of a dihydrochloride is obtained in the form of a white solid.

### Example B

### 1-[(1-Ethylhydrazino)iminomethyl]piperazine

### a) Benzylidene derivative of 1-(hydrazinoiminomethyl)piperazine

The pH of a solution of 10.7 g of the benzylidene derivative of 1-(hydrazinoiminomethyl)piperazine in the form of a dihydrochloride in 100 ml of water is adjusted to 10 by addition of 8N NaOH. The mixture obtained is extracted with ethyl acetate. The ethyl acetate phase is dried and the solvent is evaporated off. The benzylidene derivative of 1-(hydrazinoinunomethyl)piperazine is obtained in the form of an amorphous powder.

### b) Benzylidene derivative of 1-formyl-4-(hydrazinoiminomethyl)piperazine

12.7 ml of acetic acid anhydride are added dropwise to 42 ml of ice-cooled formic acid, the mixture is stirred for ca. 1 hour and 16 g of the benzylidene derivative of 1-(hydrazinoiminomethyl)piperazine in 42 ml of formic acid are added dropwise. The mixture is left for ca. 2 hours at 0° and the solvent is evaporated off. The residue is treated with water and the pH of the mixture obtained is adjusted to pH 11 by addition of 10N KOH. The mixture is extracted with dichloromethane, the dichloromethane phase is dried and the solvent is evaporated off. The benzylidene derivative of 1-formyl-4-(hydrazinoiminomethyl)piperazine is obtained in the form of a white powder.

### c) Benzylidene derivative of 1-[(1-ethylhydrazino)iminomethyl]-4-formylpiperazine

An ice-cooled solution of 2 g of the benzylidene derivative of 1-formyl-4-(hydrazinoiminomethyl)piperazine in 40 ml of dry tetrahydrofurane is treated with 9.3 ml of bis(trimethyl-silyl)-lithiumamid (1M solution in tetrahydrofurane) and stirred for ca. 1 hour at 0°. 2.4 g of ethyl iodide are added to the reaction mixture and the mixture is stirred overnight at room temperature. The solvent is evaporated off and the residue is purified via "Dry-column-flash-chromatography": Eluent: 1. methanol; 2. 90% methanol /10 % acetic acid.

Fractions containing the benzylidene derivative of 1-[(1-ethylhydrazino)iminomethyl]-4-formylpiperazine (analytical HPLC determination) are combined, the solvent is evaporated off and the benzylidene derivative of 1-[(1-ethylhydrazino)iminomethyl]-4-formylpiperazine is obtained in the form of a white powder.

### d) 1-[(1-Ethylhydrazino)iminomethyl]piperazine

2.7 g of the benzylidene derivative of 1-[(1-ethylhydrazino)iminomethyl]-4-formylpiperazine dissolved in 11.6 ml of 2N HCl are treated by steam distillation. After evaporation of the water from the mixture obtained and drying of the residue 1-[(1-ethylhydrazino)iminomethyl]piperazine in the form of a dihydrochloride is obtained in the form of a white solid.

In the manner as described in Example B, but using the corresponding reactants the following compounds may be obtained:

### Example C

1-[(1-Allylhydrazino)iminomethyl]piperazine (in the form of a dihydrochloride)

### Example D

1-[[1-(4-Methoxybenzyl)hydrazino]iminomethyl]piperazine (in the form of a dihydrochloride)

### Example E

1-[[1-(3,4,5-Trimethoxybenzyl)hydrazino]iminomethyl]piperazine (in the form of a dihydrochloride)

### Example F

### 1-[(1-Methylhydrazino)(methylimino)methyl]piperazine

### a) Benzylidene derivative of 1-formyl-4-[hydrazino(methylimino)methyl]piperazine

37 g of 1-formyl-4-[hydrazino(methylimino)methyl]piperazine in the form of a hydrochloride dissolved in a mixture of 80 ml of acetonitrile and 185 ml of water are treated with 30 g of benzaldehyde. The mixture is stirred for ca. 3 hours at room temperature and extracted with ether. The water of the aqueous phase is evaporated. The residue is treated with water and a pH of 11 of the mixture is adjusted with 2N NaOH. The mixture is extracted with dichloromethane, the organic phase is dried, the solvent evaporated and the residue is dried. The benzylidene derivative of 1-formyl-4-[hydrazino(methylimino)methyl]piperazine is obtained in the form of a white powder.

### b) Benzylidene derivative of 1-formyl-4-[(1-methylhydrazino)(methylimino)methyl]piperazine

A solution of 1,62 g of the benzylidene derivative of 1-formyl-4-[hydrazino(methylimino)-methyl]piperazine in 30 ml of acetonitrile is treated with 4,56 g of methyl iodide and the mixture is refluxed overnight. The solvent is evaporated off and the residue is stirred with 20 ml of water and 10 ml Amberlite IRA-400 (Cl)^{R} (ion exchange resin) for ca. 1 hour at room temperature. The mixture is filtrated. The aqueous solution is adjusted to a pH of 11 with 2N NaOH and extracted with dichloromethane. The organic phase is dried and concentrated by solvent evaporation. For purification the concentrate is treated in the manner as described in Example B, c).The benzylidene derivative of 1-formyl-4-[(1-methylhydrazino)(methylimino)methyl]piperazine is obtained in the form of a white powder.

### c) 1-[(1-Methylhydrazino)(methylimino)methyl]piperazine

1.14 g of the benzylidene derivative of 1-formyl-4-[(1-methylhydrazino)(methylimino)methyl]piperazine dissolved in 6 ml of 2N HCl are treated in the manner as described in Example Bd). 1-[(1-Methylhydrazino)(methylimino)methyl]piperazine in the form of a dihydrochloride is obtained in the form of a white solid.

### Example G

In the manner as described in Example F but using the corresponding reactants **1-[(1-methylhydrazino)(ethylimino)methyl]pipetazine** (in the form of a dihydrochloride) is obtained.

### Example L

### [6(R)-6α,7β(Z)]-7-[[(5-Amino-1,2,4-thiadiazo-3-yl)-(fluormethoxyimino)acetyl]amino]- 3-formyl-3-cephem-4-carboxylic acid-1-(isopropoxycarbonyloxy)ethylester

### a) [6(R)-6α,7β(Z)]-7-[[(5-Amino-1,2,4-thiadiazo]-3-yl)-(fluormethoxy-imino)acetyl]amino]-3-formyl-3-cephem-4-carboxylic acid

0.4 ml of Hünig-base are added dropwise to 1 g of N-(1,4,5a,6-Tetrahydro-3-hydroxy-1,7-dioxo-3H,7H-azeto[2,1-b]furo[3,4-d][1,3]thiazin-6-yl)-2-(5-amino-1,2,4-thiadiazol-3-yl)-(Z)-2-(fluormethoxyimino)acetic acid amide in 76 ml of acetonitrile. The solution obtained is treated with 0.38 g of sodium iodide dissoluted in 5 ml of acetonitrile. [6(R)-6α,7β(Z)]-7-[[(5-Amino-1,2,4-thiadiazol-3-yl)-(fluormethoxy-imino)acetyl]amino]-3-formyl-3-cephem-4-carboxylic acid in the form of a sodium salt precipitates, is filtrated off and dried.

### b) [6(R)-6α,7β(Z))-7-[[(5-Amino-1,2,4-thiadiazol-3-yl)-fluormethoxyimino)acetyl]amino]-3-formyl-3-cephem-4-carboxyylic acid-1-(isopropoxycarbonyloxy)ethylester

1 g of [6(R)-6α,7β(Z)]-7-[[(5-Amino-1,2,4-thiadiazol-3-yl)-(fluormethoxy-imino)acetyl]amino]-3-formyl-3-cephem-4-carboxylic acid in the form of a sodium salt in 10 ml of dimethyl-acetamide is treated at 0° under stirring with a solution of 0,65 g of 1-iodoethylisopropylcar-bonate in 4 ml of toluene and the mixture obtained is stirred for ca. 90 minutes at 0°. The mixture obtained is diluted with 100 ml of ethyl acetate and extracted with an aqueous potassium hydrogencarbonate solution. The organic phase is extracted with water, dried over NA₂SO₄ and concentrated to a volume of 10 ml. The concentrate obtained is introduced into 120 ml of n-hexane. A mixture of two diastereoisomers in a ratio of ca. 1:1 of [6(R)-6α,7β(Z))-7-[[(5-Amino-1,2,4-thiadiazol-3-yl)-fluormethoxyimino)-acetyl]amino]-3-formyl-3-cephem-4-carboxyylic acid-1-(isopropoxycarbonyloxy)ethylester precipitates, is filtrated off, dried and obtained in form of a solid.

### ¹H-NMR-Spectra

Ex.
- **1:**: 3,25 (broad, 4H, -CH₂-N-CH₂-); 3,3 (s, 3H, N-CH₃); 3,60 and 4,28 (AB quartet, J = 18 Hz, 2H, SCH2); 3,74 (broad, 4H, -CH2-NH+-CH2-); 5,28 (d, J = 5 Hz, 1H, β-lactam-H); 5,78 (d, J = 55 Hz, 2H, CH₂F); 5,91 (dd, J = 5 and 8,3 Hz, 1H, β-lactam-H); 8,1 (s, 1H, CH=N); 9,04 (broad singulet, 1H, NH); 9,35 (broad singulet, 1H, NH); (9,81 (d, J = 8,3 Hz, 1H, NH), 9,9 (broad singulet, 2H, NH₂).
- **2:**: 1.17, t, J=5 Hz, 3H, CH₃; 3.28, b, 4H, N-CH₂; 3.60 and 4.21, AB-quartet, J=18 Hz, 2H, S-CH₂; 3.67, b, 4H, N-CH₂, 3.91, m, 2H, CH₂; 5.22, d, J=5 Hz, 1H, β-lactam-H, 5.82, d, J=55 Hz, 2H, CH₂F; 5.85, dd, J= 5Hz and 8 Hz, 1H, β-lactam-H; 8.35, b, 3H, 1H CH=N and 2H, NH; 9.78, d, J=8 Hz, 1H, NH.
- **3:**: 1.18, t, J=5 Hz, 3H, CH₃; 3.30, b, 9H, 4H of NCH₂ and 2H of CH₂ and 3H of CH₃; 3.70, m, 5H, 4H of NCH₂ and 1H of S-CH₂; 4.10, part of AB-quartet, J=18 Hz, 1H, SCH₂; 5.32, d, J=5 Hz, 1H, β-lactam-H; 5.82, d, J=55 Hz, 2H, CH₂F; 5.95, dd, J=5 Hz and 8Hz, 1H, β- lactam-H; 8.08, s, 1H, CH=N; 8.32, b, 1H, NH; 9.82, d, J=8 Hz, 1H, NH.
- **4:**: 3.30, b, 4H, N-CH₂; 3.58 and 4.25, AB-quartet, J=18 Hz, 2H, S-CH₂; 3.73, b, 4H, N-CH₂; 4.30, m, 2H, N-CH₂; 5.26, m, 3H, 1H β-lactam-H and 2H of CH₂=C; 5.64, part of dublet, 1H, CH₂F; 5.90, m, 4H, 1H of CH₂-F and 1H of CH=C adn 1H β-lactam-H; 8.11, s, 1H, CH=N; 9.81, d, J=8 Hz, 1H, NH.
- **5:**: 90 and03, 2s (2:1), 3H, N-3-CH₃; 3.33, b, 7H, 4H of-CH₂ and H₃, 3.64, b, 5H, 4H of NH₂and 1H of S-CH₂; 4.15, part of AB-quartet; J=18 Hz, 1H, S-CH₂; 5.21, d, J=5 Hz, 1H, β-lactam-H; 5.81, d, J=55 Hz, 2H, CH₂F; 5.83, dd, J=5Hz and J=8 Hz, 1H, β-lactam-H; 8.32, 3H, 1H of CH=N and 2H of NH; 9.79, d, J=8 Hz, 1H, NH.
- **6:**: 3.31, b, 4H, N-CH₂; 3.52 and 4.18, AB-quartet, J=18 Hz, 2H, S-CH₂, 3.72, b, 7H, 4H of N-CH₂ and 3H of OCH₃; 4.95, AB-quartet, J=17 Hz, 2H, CH₂; 5.14, d, J=5 Hz, 1H, β-lactam-H; 5.78, d, J=55 Hz, 2H, CH₂F; 5.77, dd, J=5 Hz and 8 Hz, 1H, β-lactam-H; 6.86 - 6.91, m, 2H, CH- arom.; 7.15 - 7.19, m, 2H, CH-arom.; 8.26, b, 2H, CH=N and NH; 8.40, b, 1H, NH; 9.74, d, J=8 Hz; 1H, NH.
- **7:**: 3.34, b, 4H, N-CH₂; 3.57 and 4.23, AB-quartet, J=18 Hz, 2H, S-CH₂; 3.64, s, 3H, OCH₃; 3.79, b, 10H, 4H of N-CH₂ and 6H of OCH₃; 5.03, AB-quartet, J=17 Hz, 2H, CH2; 5.27, d, J=5 Hz, 1H, β-lactam-H; 5.81, d, J=55 Hz, 2H, CH₂F; 5.92, dd, J=5 Hz and 8 Hz, 1H, β-lactam-H; 6.53, s, 2H, CH-arom.; 8.14, s, 1H, CH=N; 8.30, b, 2H, NH; 9.83, d, J=8 Hz, 1H, NH.
- **Aa:**: 2,55 (s, 3H, S-CH₃); 3,45 (s, 3H, N-CH₃).
- **Ab:**: 3,4 (s, 3H, N-CH₃); 3,51 (m, 2H) and 3,58 (m, 6H, -CH₂-N-CH₂-); 7,45 - 7,48 (m, 3H, CH arom.); 7,81 - 7,85 (m, 2H, CH arom.); 8,10 (s, 1H, N-CH=O); 8,14 (s, 1H, CH=N); 9,0 (broad singulet, 2H, N⁺H₂).
- **Ac:**: 3,16 (m, 7H, N-CH₃ and -CH₂-N-CH₂-); 3,61 (m, 4H, -CH₂-N⁺-CH₂-); 6.0 (broad singulet, 3H, N⁺H₃); 8,3 (broad singulet, 1H, NH); 10.0 (broad singulet, 2H, N⁺H₂).
- **B:**: 1.22, t, J=5 Hz, 3H, CH₃; 3.16, b, 4H, N-CH₂; 3.45, q, J=5 Hz, 2H, CH₂; 3.65, b, 4H, N-CH₂; 10.14, b, 2H, NH.
- **C:**: 3.14, b, 4H, N-CH₂; 3.68, b, 4H, N-CH₂; 3.98 - 4.18, m, 2H, CH₂-C; 5.16 - 5.48, m, 2H, CH₂=C; 5.80 - 6.10, m, 1H, CH=C; 10.30, b, 2H, NH.
- **D:**: 3.19, b, 4H, N-CH₂; 3.67, b, 4H, N-CH₂; 3.77, s, 3H, O-CH₃; 4.59, s, 2H, N-CH₂; 6.90 - 7.02 and 7.25 - 7.38, m, each 2H, CH-arom.; 10.02, b, 2H, NH.
- **E:**: 3.20, b, 4H, N-CH₂; 3.67, b, 7H, 4H of N-CH₂ and 3H of O-CH₃; 3.81, s, 6H, O-CH₃; 4.59, s, 2H, N-CH₂; 6.69, s, 2H, CH-arom.; 9.96, b, 2H, NH.
- **F:**: 2.84, s, 3H, CH₃; 3.18, b, 7H, 4H of N-CH₂ and 3H of CH₃; 3.63, b, 4H, N-CH₂; 10.13, b, 2H, NH.
- **G:**: 1.20, t, J=5 Hz, 3H, CH₃; 3.19, b, 9H, 4H of N-CH₂ and 3H of CH₃ and 2H of CH₂; 3.64, b, 4H, N-CH₂; 10.12, b, 2H, NH.
- **La:**: 3.32 and 3.70 (AB Quartet, J = 17 Hz, 2H, SCH₂); 5.22 (d, J = 5 Hz, 1H, β-lactam-H); 5.82 (d, J = 55 Hz, 2H, CH₂F); 5.86 (dd, J = 5 and 8,4 Hz, 1H, β-lactam-H); 8.35 (broad singulet, 2H, NH₂); 9.5 (s, 1H, CH=O); 9.88 (d, J = 8,4 Hz, 1H, NH).
- **Lb:**: Diastereomer A: 1.21 (d, J = 6 Hz, 6H); 1.53 (d, J = 5.4 Hz, 3H, -O(CH₃)CH-O-); 3.67 (AB-quartet, J = 18.2 Hz, S-CH₂); 4.6-4.9 (m, 2 H, -O-CH(CH₃)₂; 5.32 (d, J = 5.3 Hz, 1H, β-lactam-H); 5.8 (d, J = 55 Hz, 2H, CH₂F); 6.04 (dd, J = 5.3 and 8.4 Hz, 1H, β-lactam-H); 6.84 (q, 1H, O(CH₃)CH-O-); 8.2 (broad singulet, 2H, NH₂); 9.6 (s, 1H, CH=O); (broad singulet, 2H, NH₂); 9.88 (d, J = 8,4 Hz, 1H, NH).
Diastereomer B: 1.23 (d, J = 6 Hz, 6H); 1.53 (d, J = 5.4 Hz, 3H, -O(CH₃)CH-O-); 3.68 (AB-quartet, J = 18.2 Hz, S-CH₂); 4.6-4.9 (m, 1H, -O-CH(CH₃)₂; 5.33 (d, J = 5.3 Hz, 1H, β-lactam-H); 5.8 (d, J = 55 Hz, 2H, CH₂F); 6.08 (dd, J = 5.3 and 8.4 Hz, 1H, β-lactam-H); 6.93 (q, 1H, O(CH₃)CH-O-); 9.6 (s, 1H, CH=O): 9.88 (d, J = 8.4 Hz, 1H, NH).

## Claims

1. A compound of formula 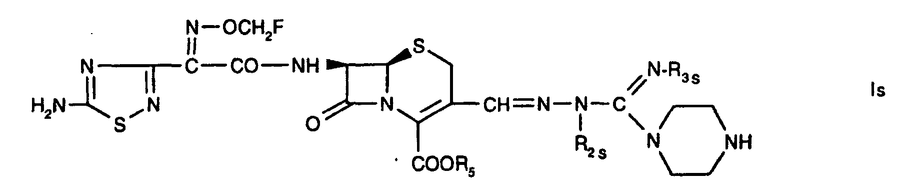 wherein
R₅ denotes hydrogen, (C₁₋₆)alkyl, benzyl, (C₁₋₈)alkoxybenzyl, indanyl, phtalidyl, (C₁₋₈)alkoxymethyl, (C₁₋₆)alkanoyloxy(C₁₋₆)alkyl, (C₁₋₆)alkoxycarbonyloxy(C₁₋₆)alkyl, glycyloxymethyl, phenylglycyloxymethyl or (5-methyl-2-oxo-1,3-dioxolen-4-yl)methyl ;
R₂ₛ denotes (C₁₋₆)alkyl, ar(C₁₋₆)alkyl, (C₂₋₆)alkenyl or (C₂₋₈)alkinyl, and
R₃ₛ denotes hydrogen, (C₁₋₆)alkyl, ar(C₁₋₆)alkyl, (C₂₋₆)alkenyl, (C₂₋₈)alkinyl or (C₃₋₈)cycloalkyl, in free form or in form of a salt or in form of a solvate.

2. A compound according to claim 1 wherein R₂ₛ is methyl and R₃ₛ is hydrogen.

3. A compound according to claims 1 or 2 which is 7-(((5-Amino-1,2,4-thiadiazol-3-yl)-(Z)-(fluoromethoxyimino)acetyl)amino)-3(E)-((imino-1-piperazinylmethyl)methylhydrazono)methyl-3-cephem-4-carboxylic acid in the form of a hydrochloride.

4. A compound according to claims 1 or 2 which is 7-(((5-Amino-1,2,4-thiadiazol-3-yl)-(Z)-(fluoromethoxyimino)acetyl)amino)-3(E)-((imino-1-piperazinylmethyl)methylhydrazono)methyl-3-cephem-4-carboxylic add in the form of a trihydrochloride.

5. A compound selected from the group consisting of
1-[(1-Ethylhydrazino)iminomethyl]piperazine,
1-[(1-Allylhydrazino)iminomethyl]piperazine,
1-[(1-(4-Methoxybenzyl)hydrazino]iminomethyl]piperazine,
1-[(1-(3,4,5-Trimethoxybenzyl)hydrazino]iminomethyl]piperazine,
1-[(1-Methylhydrazino)(methylimino)methyl]piperazine, and
1-[(1-Methylhydrazino)(ethylimino)methyl]piperazine.

6. Use of a compound of claim 5 in the production of a compound of formula Is as defined in claim 1.

7. A compound of formula 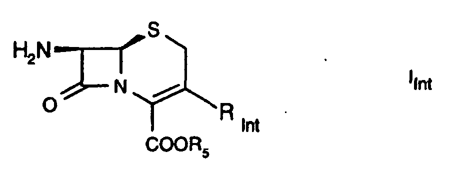 wherein R₅ denotes hydrogen, (C₁₋₆)alkyl, benzyl, (C₁₋₈)alkoxybenzyl, indanyl, phtalidyl, (C₁₋₈)alkoxymethyl, (C₁₋₆)alkanoyloxy (C₁₋₆)alkyl, (C₁₋₆)alkoxycarbonyloxy(C₁₋₆)alkyl, glycyloxymethyl, phenylglycyloxymethyl or (5-methyl-2-oxo-1,3-dioxolen-4-yl)methyl, and Rᵢₙₜ denotes a group 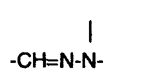 wherein the =N- group corresponds to the terminal amine group of the hydrazino group of a compound of claim 5 and wherein the -N〈 group is substituted according to a compound of claim 5.

8. Use of a compound of formula I_{Int} as defined in claim 7 in the production of a compound of formula Is as defined in claim 1

9. A process for the production of a compound of formula Is, as defined in claim 1, comprising reacting a compound of formula 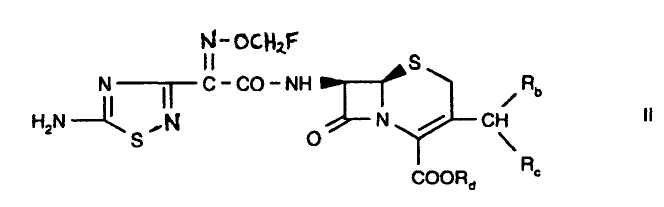 wherein either
α) R_{b} denotes hydroxy and R_{c} and R_{d} together denote a bond, or
β) R_{d} denotes hydrogen, a cation, (C₁₋₆)alkyl, benzyl, (C₁₋₈)alkoxybenzyl, indanyl, phtalidyl, (C₁₋₈)alkoxymethyl, (C₁₋₆)alkanoyloxy(C₁₋₆)alkyl, (C₁₋₆)alkoxycarbonyloxy(C₁₋₆)alkyl, glycyloxymethyl, phenylglycyloxymethyl or (5-methyl-2-oxo-1,3-dioxolen-4-yl)methyl or a silyl protecting group and R_{b} and R_{c} together denote the oxo group
with a compound of formula 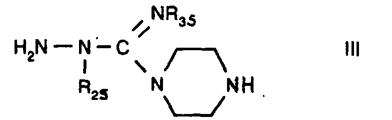 wherein R₂ₛ and R₃ₛ are as defined as in claim 1 and isolating a compound of formula Is.

10. A pharmaceutical composition comprising a compound of formula Is as defined in claim 1 in the form of a pharmaceutically acceptable salt or in free form in association with at least one pharmaceutical carrier or diluent.

11. Use of a compound of formula Is as defined in any one of claims 1 to 4 in the preparation of a medicament for treating bacterial diseases caused by a bacteria genus selected from the group consisting of *Pseudomonas, Enterobocter, Enterococcus, Moraxella, Haemophilus, Klebsiella, Streptococcus, Staphylococcus, Escherichia, Proteus, Salmonella, Serratia* and *Pneumococcus*.

## Patentansprüche

1. Verbindung der Formel 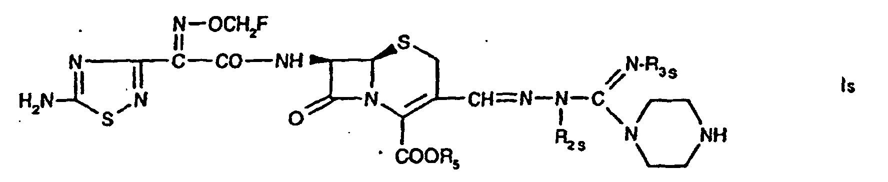 worin
R₅ für Wasserstoff, (C₁-C₆)Alkyl, Benzyl, (C₁-C₈)Alkoxybenzyl, Indanyl, Phthalidyl, (C₁-C₈)Alkoxymethyl, (C₁-C₆)Alkanoyloxy(C₁-C₆)alkyl, (C₁-C₆)Alkoxycarbonyloxy(C₁-C₆)alkyl, Glycyloxymethyl, Phenylglycyloxymethyl oder (5-Methyl-2-oxo-1,3-dioxolen-4-yl)methyl steht,
R₂ₛ für (C₁-C₆)Alkyl, Ar(C₁-C₆)alkyl, (C₂-C₆)Alkenyl oder (C₂-C₈)Alkinyl steht und
R₃ₛ für Wasserstoff, (C₁-C₆)Alkyl, Ar(C₁-C₆)alkyl, (C₂-C₆)Alkenyl, (C₂-C₈)Alkinyl oder (C₃-C₈)Cycloalkyl steht in freier Form oder in Form eines Salzes oder in Form eines Solvats.

2. Verbindung nach Anspruch 1, worin R₂ₛ für Methyl steht und R₃ₛ für Wasserstoff steht.

3. Verbindung nach Anspruch 1 oder 2, nämlich -(fluormethoxyimino)acetyl)amino)-3(E)-((imino-1-piperazinylmethyl)methylhydrazono)methyl-3-cephem-4-carbonsäure in Form eines Hydrochlorids.

4. Verbindung nach Anspruch 1 oder 2, nämlich 7-((5-Amino-1,2,4-thiadiazol-3-yl)-(Z)-(fluormethoxyimino)acetyl)amino)-3(E)-((imino-1-piperazinylmethyl)methylhydrazono)methyl-3-cephem-4-carbonsäure in Form eines Trihydrochlorids.

5. Verbindung, die aus der Gruppe ausgewählt ist, welche besteht aus
1-[(1-Ethylhydrazino)iminomethyl]piperazin,
1-[(1-Allylhydrazino)iminomethyl]piperazin,
1-[(1-(4-Methoxybenzyl)hydrazino]iminomethyl]piperazin,
1-[(1-(3,4,5-Trimethoxybenzyl)hydrazino]iminomethyl]piperazin,
1-[(1-Methylhydrazino)(methylimino)methyl]piperazin und
1-[(1-Methylhydrazino)(ethylimino)methyl]piperazin.

6. Verwendung einer Verbindung nach Anspruch 5 zur Herstellung einer Verbindung der Formel Is gemäß Definition von Anspruch 1.

7. Verbindung der Formel 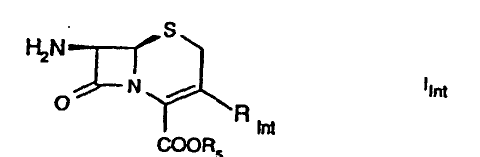 worin R₅ für Wasserstoff, (C₁-C₆)Alkyl, Benzyl, (C₁-C₈)Alkoxybenzyl, Indanyl, Phthalidyl, (C₁-C₈)Alkoxymethyl, (C₁-C₆)Alkanoyloxy(C₁-C₆)alkyl, (C₁-C₆)Alkoxycarbonyloxy(C₁-C₆)alkyl, Glycyloxymethyl, Phenylglycyloxymethyl oder (5-Methyl-2-oxo-1,3-dioxolen-4-yl)methyl steht und Rᵢₙₜ für die folgende Gruppe steht 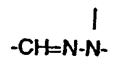 worin die Gruppe =N- der tetininalen Amingruppe der Hydrazinogruppe einer Verbindung nach Anspruch 5 entspricht und worin die Gruppe 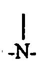 gemäß einer Verbindung von Anspruch 5 substituiert ist

8. Verwendung einer Verbindung der Formel Iᵢₙₜ gemäß Definition von Anspruch 7 zur Herstellung einer Verbindung der Formel Is gemäß Definition von Anspruch 1.

9. Verfahren zur Herstellung einer Verbindung der Formel Is gemäß Definition von Anspruch 1, **gekennzeichnet durch** Umsetzung einer Verbindung der Formel 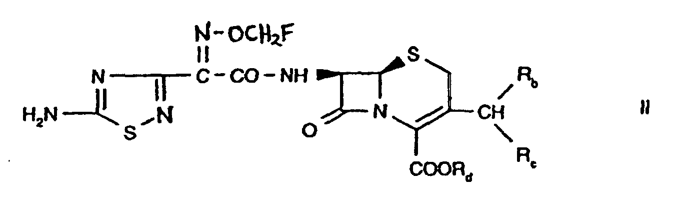 worin entweder
α) R_{b} für Wasserstoff steht und R_{c} und R_{d} zusammen eine Bindung bedeuten oder
β) R_{d} für Wasserstoff, ein Kation, (C₁-C₆)Alkyl, Benzyl, (C₁-C₈)Alkoxybenzyl, Indanyl, Phthalidyl, (C₁-C₈)Alkoxymethyl, (C₁-C₆)Alkanoyloxy(C₁-C₆)alkyl, (C₁-C₆)Alkoxycarbonyloxy(C₁-C₆)alkyl, Glycyloxymethyl, Phenylglycyloxymethyl oder (5-Methyl-2-oxo-1,3-dioxolen-4-yl)methyl oder eine Silylschutzgruppe steht und R_{b} und R_{c} zusammen für die Oxogruppe stehen,
mit einer Verbindung der Formel 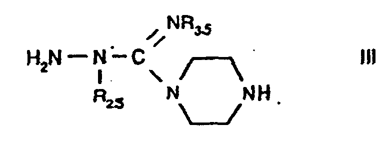 worin R₂ₛ und R₃ₛ wie im Anspruch 1 definiert sind, und Isolierung einer Verbindung der Formel Is.

10. Pharmazeutische Zusammensetzung, enthaltend eine Verbindung der Formel Is gemäß Definition von Anspruch 1 in Form eines pharmazeutisch annehmbaren Salzes oder in freier Form in Assoziation mit wenigstens einem pharmazeutischen Träger oder Verdünnungsmittel.

11. Verwendung einer Verbindung der Formel Is gemäß Definition nach einem der Ansprüche 1 bis 4 zur Herstellung eines Arzneimittels zur Behandlung bakterieller Erkrankungen, die durch eine Bakteriengattung hervorgerufen werden, welche aus der Gruppe ausgewählt ist, die besteht aus Pseudomonas, Enterobacter, Enterococcus, Moraxella, Haernophilus, Klebsiella, Streptococcus, Staphylococcus, Escherichia, Proteus, Salmonella, Serratia und Pneumococcus.

## Revendications

1. Composé de formule : 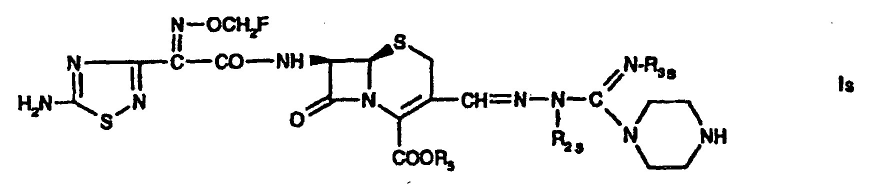 dans laquelle
R₅ représente l'hydrogène, un groupe alkyle en C₁ à C₆, benzyle, alcoxy(en C₁ à C₈)benzyle, indanyle, phtalidyle, alcoxy(en C₁ à C₈)méthyle, alcanoyloxy en C₁ à C₆-alkyle en C₁ à C₆, alcoxy(en C₁ à C₆)carbonyloxyalkyle en C₁ à C₆, glycyloxyméthyle, phénylglycyloxyméthyle ou (5-méthyl-2-oxo-1,3-dioxolén-4-yl)méthyle ;
R_{2S} représente un groupe alkyle en C₁ à C₆, ar-(alkyle en C₁ à C₆), alcényle en C₂ à C₆ ou alcynyle en C₂ à C₈ ; et
R_{3S} représente l'hydrogène, un groupe alkyle en C₁ à C₆, ar-(alkyle en C₁ à C₆), alcényle en C₂ à C₆, alcynyle en C₂ à C₈ ou cycloalkyle en C₃ à C₈,
sous forme libre, sous forme d'un sel ou sous forme d'un produit de solvatation.

2. Composé suivant la revendication 1, dans lequel R_{2S} représente un groupe méthyle et R_{3S} représente l'hydrogène.

3. Composé suivant la revendication 1 ou 2, qui est l'acide 7-(((5-amino-1,2,4-thiadiazol-3-yl)-(Z)-(fluorométhoxyimino)acétyl)amino)-3(E)-((imino-1-pipérazinylméthyl)méthylhydrazono)méthyl-3-céphème-4-carboxylique sous forme d'un chlorhydrate.

4. Composé suivant la revendication 1 ou 2, qui est l'acide 7-(((5-amino-1,2,4-thiadiazol-3-yl)-(Z)-(fluorométhoxyimino)acétyl)amino)-3(E)-((imino-1-pipérazinylméthyl)méthylhydrazono)méthyl-3-céphème-4-carboxylique sous forme d'un trichlorhydrate.

5. Composé choisi dans le groupe constitué par :
la 1-[(1-éthylhydrazino)iminométhyl]pipérazine,
la 1-[(1-allylhydrazino)iminométhyl]pipérazine,
la 1-[(1-(4-méthoxybenzyl)hydrazino)iminométhyl]pipérazine,
la 1-[(1-(3,4,5-triméthoxybenzyl)hydrazino]iminométhyl]pipérazine,
la 1-[(1-méthylhydrazino)(méthylimino)méthyl]pipérazine, et
la 1-[(1-méthylhydrazino)(éthylimino)méthyl]pipérazine.

6. Utilisation d'un composé suivant la revendication 5 pour la fabrication d'un composé de formule Is de la manière indiquée dans la revendication 1.

7. Composé de formule : 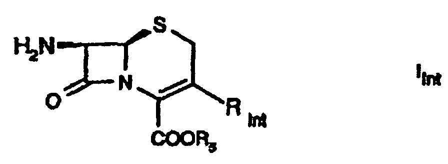 dans laquelle R₅ représente l'hydrogène, un groupe alkyle en C₁ à C₆, benzyle, alcoxy(en C₁ à C₈)benzyle, indanyle, phtalidyle, alcoxy(en C₁ à C₈)méthyle, alcanoyloxy en C₁ à C₆-alkyle en C₁ à C₆, alcoxy(en C₁ à C₆)carbonyloxyalkyle en C₁ à C₆, glycyloxyméthyle, phénylglycyloxyméthyle ou (5-méthyl-2-oxo-1,3-dioxolén-4-yl)méthyle, et Rᵢₙₜ représente un groupe : 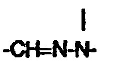 dans lequel le groupe =N⁻ correspond au groupe amine terminal du groupe hydrazino d'un composé suivant la revendication 5, et dans lequel le groupe -N〈 est substitué conformément à un composé suivant la revendication 5.

8. Utilisation d'un composé de formule I_{Int} tel que défini dans la revendication 7 dans la fabrication d'un composé de formule Is tel que défini dans la revendication 1.

9. Procédé de fabrication d'un composé de formule Is, tel que défini dans la revendication 1, comprenant la réaction d'un composé de formule : 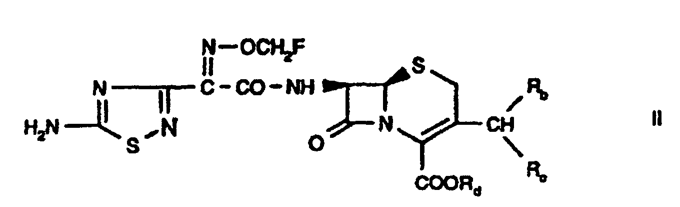 dans laquelle soit
α) R_{b} représente un groupe hydroxy et R_{c} et R_{d} représentent ensemble une liaison, soit
β) R_{d} représente l'hydrogène, un cation, un groupe alkyle en C₁ à C₆, benzyle, alcoxy(en C₁ à C₈)benzyle, indanyle, phtalidyle, alcoxy(en C₁ à C₈)méthyle, alcanoyloxy en C₁ à C₆-alkyle en C₁ à C₆, alcoxy(en C₁ à C₆)carbonyloxyalkyle en C₁ à C₆, glycyloxyméthyle, phénylglycyloxyméthyle ou (5-méthyl-2-oxo-1,3-dioxolén-4-yl)méthyle ou un groupe protecteur silyle, et R_{b} et R_{c} représentent ensemble un groupe oxo,
avec un composé de formule : 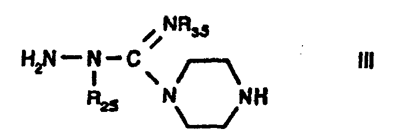 dans laquelle R_{2S} et R_{3S} sont tels que définis dans la revendication 1, et l'isolement d'un composé de formule Is.

10. Composition pharmaceutique comprenant un composé de formule Is tel que défini dans la revendication 1, sous forme d'un sel pharmaceutiquement acceptable ou sous forme libre en association avec au moins un véhicule ou diluant pharmaceutique.

11. Utilisation d'un composé de formule Is tel que défini dans l'une quelconque des revendications 1 à 4 pour la préparation d'un médicament pour le traitement de maladies bactériennes provoquées par un genre bactérien choisi dans le groupe constitué par *Pseudomonas, Enterobacter, Enterococcus, Moraxella, Haemophilus, Klebsiella, Streptococcus, Staphylococcus, Escherichia, Proteus, Salmonella, Serratia* et *Pneumococcus.*
